# EUROPEAN PATENT SPECIFICATION

(11) **EP 3 174 523 B1**
(45) Date of publication and mention of the grant of the patent: **01.07.2020**
(21) Application number: 15748420.5
(22) Date of filing: 30.07.2015
(51) Int. Cl.: A61K 9/00, A61K 38/48, A61K 47/10, A61K 47/36

(54) **FORMULATIONS OF BIOLOGICS FOR INTRAVESICAL INSTILLATION**
FORMULIERUNGEN VON BIOLOGISCHEN STOFFEN ZUR INTRAVESIKALEN INSTILLATION
FORMULATIONS D'AGENTS BIOLOGIQUES POUR INSTILLATION INTRAVÉSICALE

(30) Priority: 31.07.2014 US 201462031302 P
(43) Date of publication of application: 07.06.2017
(73) Proprietor: ALLERGAN, INC., Irvine, CA 92612 (US)
(72) Inventor: FORSSEN, Eric A., La Canada, California 91011 (US); HUGHES, Patrick M., Aliso Viejo, California 92656 (US); RUPP, David C., San Pedro, California 90731 (US)
(74) Representative: Hoffmann Eitle
(86) International application number: PCT/US2015/042988
(87) International publication number: WO 2016/019180

(56) References cited:
- WO-A1-2010/090677
- WO-A2-2013/153550
- GIANNANTONI A ET AL: "New Frontiers in Intravesical Therapies and Drug Delivery", EUROPEAN UROLOGY, ELSEVIER BV, NL, vol. 50, no. 6, 1 December 2006 (2006-12-01), pages 1183-1193, XP027882214, ISSN: 0302-2838 [retrieved on 2006-12-01]
- JAN BARTHELMES ET AL: "Development of a mucoadhesive nanoparticulate drug delivery system for a targeted drug release in the bladder", INTERNATIONAL JOURNAL OF PHARMACEUTICS, ELSEVIER BV, NL, vol. 416, no. 1, 17 June 2011 (2011-06-17) , pages 339-345, XP028264546, ISSN: 0378-5173, DOI: 10.1016/J.IJPHARM.2011.06.033 [retrieved on 2011-06-24]
- KHUTORYANSKIY VITALIY V: "Advances in mucoadhesion and mucoadhesive polymers.", MACROMOLECULAR BIOSCIENCE, vol. 11, no. 6, 14 June 2011 (2011-06-14), pages 748-764, XP002745782, ISSN: 1616-5195

## Description

### FIELD

The present disclosure relates to pharmaceutical formulations comprising a clostridial derivative and methods of use thereof. In particular, the present disclosure relates to pharmaceutical formulations containing a clostridial derivative for bladder instillation.

### BACKGROUND

Neurotoxin therapies, in particular botulinum toxins, have been used in treatments of various medical conditions, including urological conditions such as overactive bladder (OAB) and detrusor overactivity.

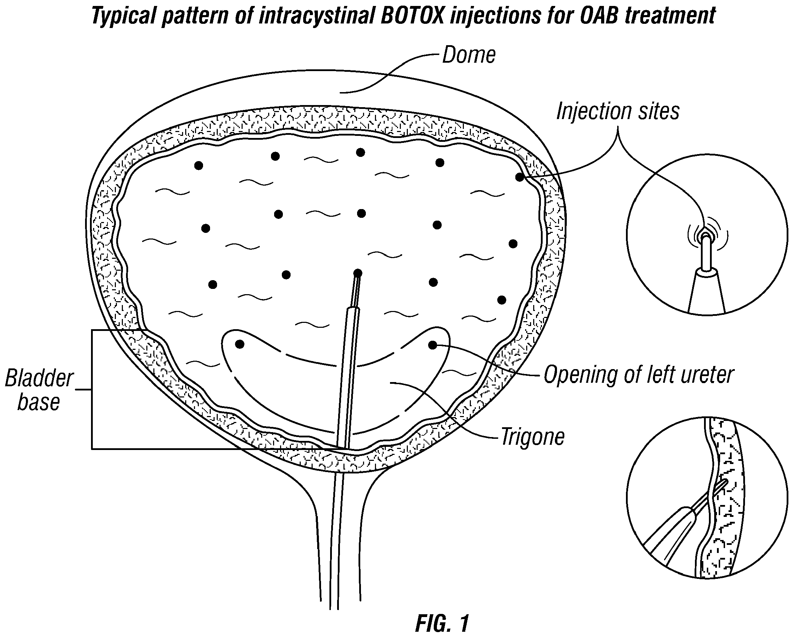

Botulinum toxin therapy to treat bladder disorders such as overactive bladder (OAB), detrusor overactivity associated with a neurological condition, is typically administered by injection across the urinary bladder wall and into the enervated muscular tissues surrounding the bladder. This approach requires administering about thirty to forty injections through the bladder wall, as shown in Figure 1. Pharmaceutical administration by injection may cause localized pain, and potentially expose patients to blood borne diseases. Among alternative administration routes, intravesical instillation allows a drug to be delivered directly into the bladder by crossing the bladder wall.

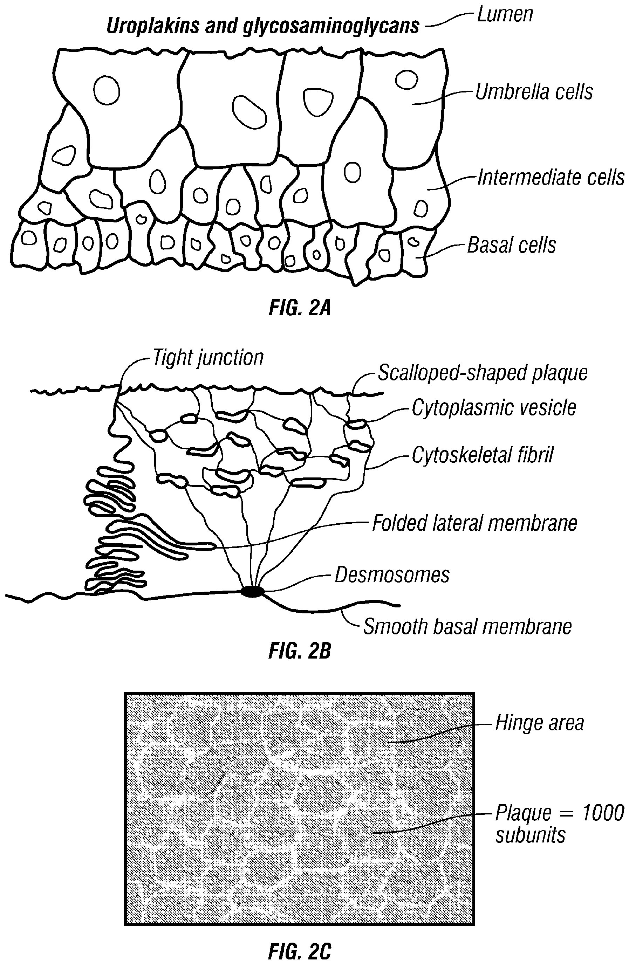

The bladder wall is impermeable to most substances. As shown in Figure 2, the stratified urothelium consists of three cellular layers: umbrella cells, intermediate cells, and basal cells. The basal cells are germinal cells that through cell division replace intermediate cells that are partially differentiated. The highly differentiated and polarized umbrella cells are located on the lumen of the bladder and are the primary physical barrier to the movement of substances between the blood and urine. The apical membrane of the umbrella cells is covered with plaques consisting of proteins called uroplakins and gives the apical membrane a thick appearance. The umbrella cells also contain tight junctions that restrict the paracellular movement of urine and larger molecules through the epithelium.

WO2013/153550 discloses compositions comprising a mucoadhesive polymer and optionally a penetration enhancer for treating bladder disorders.

Thus, there remains a need for pharmaceutical formulations that can enhance delivery across the urinary bladder wall in lieu of parenteral administration.

### SUMMARY OF THE INVENTION

In some aspects, the present disclosure provides pharmaceutical formulations for intravesical (urinary bladder) administration, comprising a clostridial derivative and at least one permeabilizing agent, which can permeate the bladder wall of a patient and retain the clostridial derivative's bioactivity to cause a desired therapeutic effect. The scope of the invention is defined by the appended claims.

In one aspect, the present disclosure provides a pharmaceutical composition comprising a therapeutically effective amount of a clostridial derivative and at least one permeabilizing agent, wherein the clostridial derivative is a botulinum toxin, and wherein the at least one permeabilizing agent comprises a non-ionic surfactant and a mucoadhesive, which is a cationic polymer, and wherein the at least one permeabilizing agent is present in an amount effective to substantially and reversibly increase the permeability of the bladder wall to the clostridial derivative.

According to another aspect, the present disclosure also provides compositions for use for treating medical disorders in a patient, as defined in claim 10. In one embodiment, the present methods alleviate one or more symptoms of the medical disorders. In one embodiment, the medical disorders include neurogenic idiopathic bladder dysfunction, or bladder pain. In some aspects, a composition for use is provided for treating a patient with a neurogenic or idiopathic bladder dysfunction, bladder pain, comprising: intravesically instilling into the bladder of the patient a pharmaceutical composition comprising a therapeutically effective amount of a clostridial derivative which is a botulinum toxin and at least one permeabilizing agent comprising a non-ionic surfactant and a mucoadhesive, which is a cationic polymer, and wherein the at least one permeabilizing agent is present in an amount effective to substantially and reversibly increase the permeability of the bladder wall to the clostridial derivative at a therapeutically effective rate. The compositions for use according to claim 10 are for use in treating a patient with a neurogenic bladder dysfunction.

### BRIEF DESCRIPTION OF THE DRAWINGS

**FIG. 1** shows a prior art intracystinal injection of a botulinum toxin to the bladder wall;
**FIGS. 2A-2C** are schematics of the urothelium, wherein:
   A) The urothelium consists of three cell layers: basal, intermediate, and superficial umbrella cells.
   B) The umbrella cells display plaques on their apical membrane and a cytoplasmic network of vesicles containing fibrils joined at the tight junction and the desmosomes on the smooth basal membrane.
   C) The apical membrane of the urothelium viewed for the lumen shows the plaque and hinge regions.

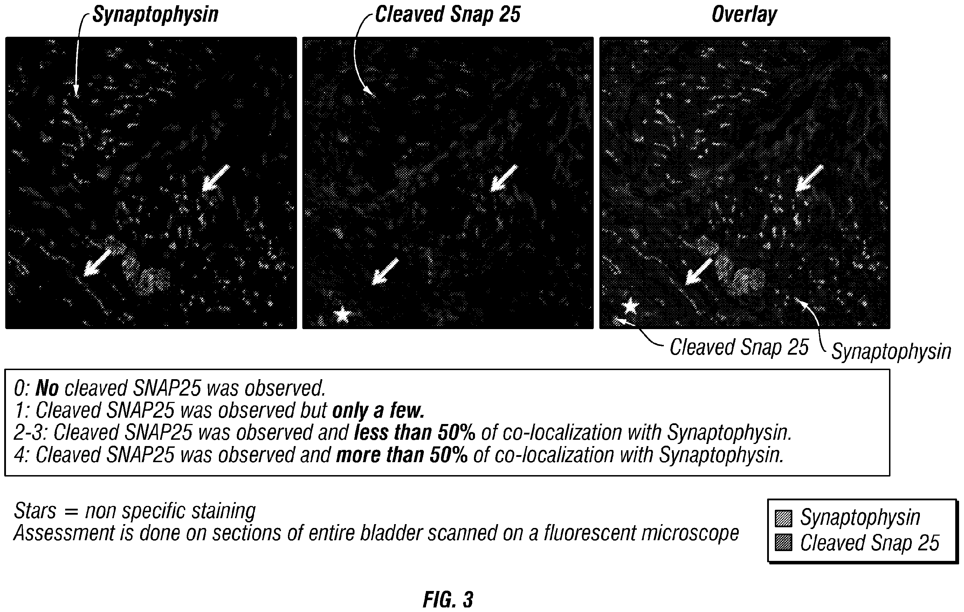

**FIG. 3** is a pictorial diagram establishing a basis for assessing the extent of penetration into the bladder of a test formulation; wherein cleaved SNAP 25 was used as a biomarker of BOTOX® activity at synaptic terminals, and Synaptophysin expression was used to identify synaptic terminals and to ensure specificity of cleaved SNAP 25 localization,. The extent of penetration was correlated to the extent of SNAP25-197 staining;

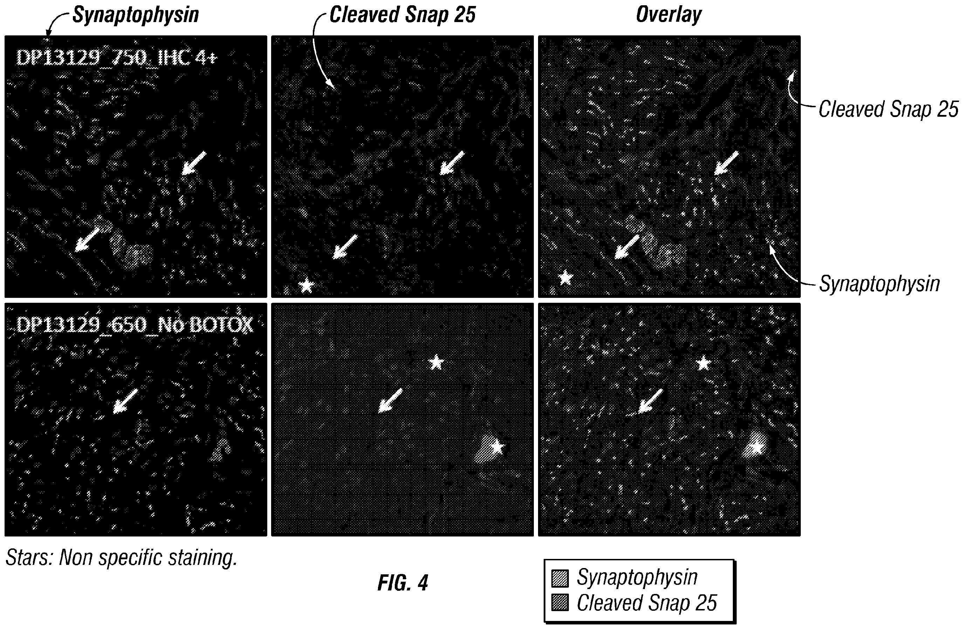

**FIG. 4** is a pictorial diagram showing exemplary immunohistochemistry results from two bladders having IHC scores of "4" and "0"; wherein cleaved SNAP 25 was used as a biomarker of BOTOX® activity at synaptic terminals, and Synaptophysin expression was used to identify synaptic terminals and to ensure specificity of cleaved SNAP 25 localization;

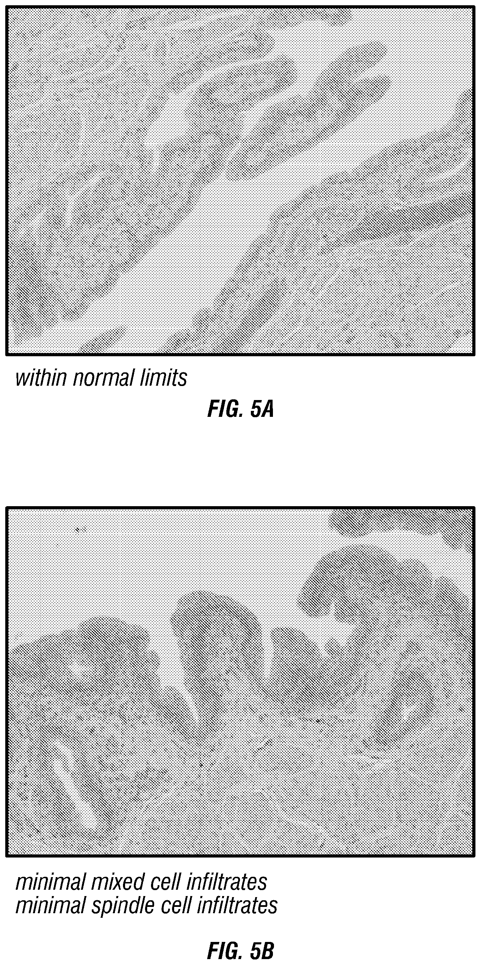

**FIGS. 5A-5D** are illustrative photomicrographs showing different states of the bladder tissue after instillation; and

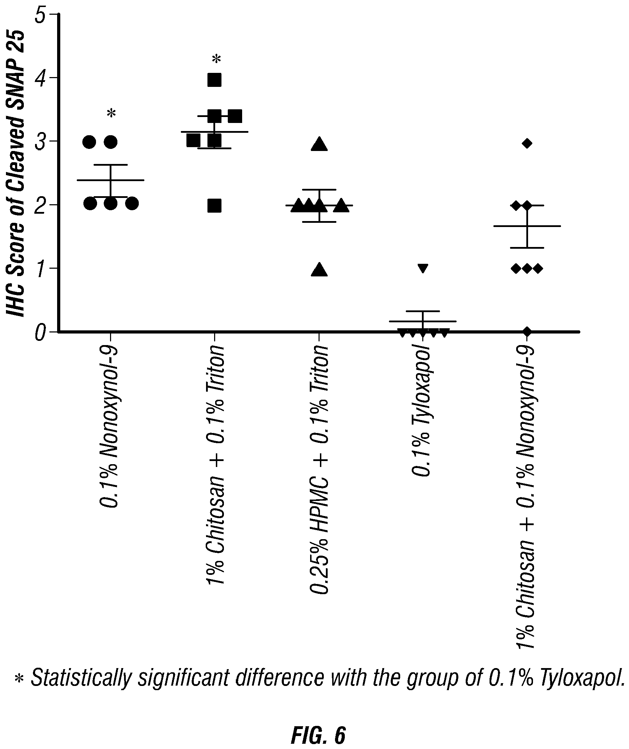

**FIG. 6** is a graph displaying the immunohistochemistry (IHC) scores of some exemplary formulations according to aspects of the present disclosure.

### DETAILED DESCRIPTION OF THE PREFERRED EMBODIMENTS

Botulinum neurotoxins (BoNTs), for example, BoNT/A, BoNT/B, etc., act on the nervous system by blocking the release of neurosecretory substances including neurotransmitters. The action of BoNT is initiated by its binding to a receptor molecule on the cell surface. The resulting toxin-receptor complex then undergoes endocytosis. Once inside the cell, BoNT cleaves exocytotic specific proteins responsible for neurotransmitter docking and release from the cell known as the SNARE proteins (soluble N-ethylmaleimide-sensitive factor attachment protein receptor). The resulting transient chemodenervation has been utilized medically to block motor neurotransmission at the neuromuscular junction, leading to a variety of therapeutic applications.
(A) One embodiment of the present disclosure provides a pharmaceutical composition comprising a therapeutically effective amount of a clostridial derivative and at least one permeabilizing agent, wherein the clostridial derivative is a botulinum toxin, and wherein the at least one permeabilizing agent comprises a non-ionic surfactant and a mucoadhesive, which is a cationic polymer, and wherein the at least one permeabilizing agent is present in an amount effective to substantially and reversibly increase the permeability of the bladder wall to the clostridial derivative.
(B) The pharmaceutical composition of embodiment (A), wherein the surfactant comprises an alkyl aryl polyether.
(C) The pharmaceutical composition of embodiment (A), wherein the mucoadhesive comprises chitosan.
(D) The pharmaceutical composition of embodiment (A), wherein the surfactant is present in an amount ranging from 0.01% to 0.5% (w/v).
(E) The pharmaceutical composition of embodiment (B), wherein the mucoadhesive is present in an amount ranging from 0.01% to 5% (w/v).
(F) The pharmaceutical composition of embodiment (B), wherein the surfactant comprises octoxynol-9.
(G) The pharmaceutical composition of embodiment (B), wherein the surfactant comprises nonoxynol-9.
(H) The pharmaceutical composition of embodiment (F), wherein the surfactant is present in an amount of 0.1 % (w/v).
(I) The pharmaceutical composition of embodiment (J), wherein the surfactant is present in an amount of 0.1% (w/v).
(J) The pharmaceutical composition according to embodiment (A) for use in treating a patient with a neurogenic bladder dysfunction, comprising: intravesically instilling to the bladder wall of the patient the above pharmaceutical composition.
(K) The pharmaceutical composition for use according to embodiment (A), wherein the surfactant comprises an alkyl aryl polyether.
(L) The pharmaceutical composition for use according to embodiment (A), wherein the mucoadhesive comprises chitosan.
(M) The pharmaceutical composition for use according to embodiment (J), wherein the surfactant is present in an amount ranging from 0.01% to 0.5% (w/v).
(N) The pharmaceutical composition for use according to embodiment (L), wherein the mucoadhesive is present in an amount ranging from 0.01% to 5% (w/v).
(O) The pharmaceutical composition for use according to embodiment (K), wherein the surfactant comprises octoxynol-9.

### Definitions

As used herein, the words or terms set forth below have the following definitions:
"Active pharmaceutical ingredient" (API) means an ingredient that exerts an effect upon or after administration to a subject or patient. API's can includea native or recombinant botulinum toxin.
"Administration", or "to administer" means the step of giving (*i.e.* administering) a pharmaceutical composition to a subject, or alternatively a subject receiving a pharmaceutical composition. The pharmaceutical compositions disclosed herein can be locally administered by various methods. For example, intramuscular, intradermal, subcutaneous administration, intrathecal administration, intraperitoneal administration, topical (transdermal), instillation, and implantation (for example, of a slow-release device such as polymeric implant or miniosmotic pump) can all be appropriate routes of administration.
"Alleviating" means a reduction in the occurrence of a pain, of a headache, of a hyperactive muscle, or of any symptom or cause of a condition or disorder. Thus, alleviating includes some reduction, significant reduction, near total reduction, and total reduction.
"Animal protein free" means the absence of blood derived, blood pooled and other animal derived products or compounds. "Animal" means a mammal (such as a human), bird, reptile, fish, insect, spider or other animal species. "Animal" excludes microorganisms, such as bacteria. Thus, an animal protein free pharmaceutical composition can include a botulinum neurotoxin. For example, an "animal protein free" pharmaceutical composition means a pharmaceutical composition which is either substantially free or essentially free or entirely free of a serum derived albumin, gelatin and other animal derived proteins, such as immunoglobulins. An example of an animal protein free pharmaceutical composition is a pharmaceutical composition which comprises or which consists of a botulinum toxin and a suitable polysaccharide as a stabilizer or excipient.
"Biological activity" describes the beneficial or adverse effects of a drug on living matter. When a drug is a complex chemical mixture, this activity is exerted by the substance's active ingredient but can be modified by the other constituents. Biological activity can be assessed as potency or as toxicity by an *in vivo* LD₅₀ or ED₅₀ assay, or through an *in vitro* assay such ascell-based potency assays as described in U.S. publications 20100203559, 20100233802, 20100233741 and U.S. Patent 8,198,034.
"Botulinum toxin" means a neurotoxin produced by Clostridium botulinum, as well as a botulinum toxin (or the light chain or the heavy chain thereof) made recombinantly by a non-Clostridial species. The phrase "botulinum toxin", as used herein, encompasses the botulinum toxin serotypes A, B, C, D, E, F and G, and their subtypes and any other types of subtypes thereof, or any re-engineered proteins, analogs, derivatives, homologs, parts, sub-parts, variants, or versions, in each case, of any of the foregoing. "Botulinum toxin", as used herein, also encompasses a "modified botulinum toxin". Further "botulinum toxin" as used herein also encompasses a botulinum toxin complex, (for example, the 300, 500 and 900kDa complexes), as well as the neurotoxic component of the botulinum toxin (150 kDa) that is unassociated with the complex proteins.
"Clostridial derivative" refers to a molecule which contains any part of a clostridial toxin. As used herein, the term "clostridial derivative" encompasses native or recombinant neurotoxins, recombinant modified toxins, fragments thereof, .
"Clostridial toxin" refers to any toxin produced by a Clostridial toxin strain that can execute the overall cellular mechanism whereby a Clostridial toxin intoxicates a cell and encompasses the binding of a Clostridial toxin to a low or high affinity Clostridial toxin receptor, the internalization of the toxin/receptor complex, the translocation of the Clostridial toxin light chain into the cytoplasm and the enzymatic modification of a Clostridial toxin substrate. Non-limiting examples of Clostridial toxins include a Botulinum toxin like BoNT/A, a BoNT/B, a BoNT/C₁, a BoNT/D, a BoNT/E, a BoNT/F, a BoNT/G,. The BoNT/C₂ cytotoxin and BoNT/C₃ cytotoxin, not being neurotoxins, are excluded from the term "Clostridial toxin." A Clostridial toxin disclosed herein includes, without limitation, naturally occurring Clostridial toxin variants, such as, e.g., Clostridial toxin isoforms and Clostridial toxin subtypes; non-naturally occurring Clostridial toxin variants, such as, e.g., conservative Clostridial toxin variants, non-conservative Clostridial toxin variants, Clostridial toxin chimeric variants and active Clostridial toxin fragments thereof, or any combination thereof. A Clostridial toxin disclosed herein also includes a Clostridial toxin complex. As used herein, the term "Clostridial toxin complex" refers to a complex comprising a Clostridial toxin and non-toxin associated proteins (NAPs), such as, *e.g*., a Botulinum toxin complex, Non-limiting examples of Clostridial toxin complexes include those produced by a *Clostridium botulinum,* such as, *e.g*., a 900-kDa BoNT/A complex, a 500-kDa BoNT/A complex, a 300-kDa BoNT/A complex, a 500-kDa BoNT/B complex, a 500-kDa BoNT/C₁ complex, a 500-kDa BoNT/D complex, a 300-kDa BoNT/D complex, a 300-kDa BoNT/E complex, and a 300-kDa BoNT/F complex.
"Effective amount" as applied to the biologically active ingredient means that amount of the ingredient which is generally sufficient to induce a desired change in the subject. For example, where the desired effect is a reduction in an autoimmune disorder symptom, an effective amount of the ingredient is that amount which causes at least a substantial reduction of the autoimmune disorder symptom, and without resulting in significant toxicity.
"Effective amount" as applied to a non-active ingredient constituent of a pharmaceutical composition (such as a stabilizer used for mixing with a botulinum toxin) refers to that amount of the non-active ingredient constituent which is sufficient to positively influence the release and/or activity of the active ingredient when administered to an individual. This "effective amount" can be determined based on the teaching in this specification and the general knowledge in the art.
"Entirely free (*i.e.* "consisting of' terminology) means that within the detection range of the instrument or process being used, the substance cannot be detected or its presence cannot be confirmed.
"Essentially free" (or "consisting essentially of') means that only trace amounts of the substance can be detected.
"Light chain" means the light chain of a clostridial neurotoxin. It has a molecular weight of about 50 kDa, and can be referred to as the L chain, L, or as the proteolytic domain (amino acid sequence) of a botulinum neurotoxin.
"Heavy chain" means the heavy chain of a botulinum neurotoxin. It has a molecular weight of about 100 kDa and can be referred to as the H chain, or as H.
   H_{C} means a fragment (about 50 kDa) derived from the H chain of a botulinum neurotoxin which is approximately equivalent to the carboxyl end segment of the H chain, or the portion corresponding to that fragment in the intact H chain. It is believed to contain the portion of the natural or wild type botulinum neurotoxin involved in high affinity, presynaptic binding to motor neurons.
   H_{N} means a fragment (about 50 kDa) derived from the H chain of a botulinum neurotoxin which is approximately equivalent to the amino end segment of the H chain, or a portion corresponding to that fragment. It is believed to contain the portion of the natural or wild type botulinum neurotoxin involved in the translocation of the L chain across an intracellular endosomal membrane.
   LH_{N} or L-H_{N} means a fragment derived from a clostridial neurotoxin that contains the L chain, or a functional fragment thereof coupled to the H_{N} domain It can be obtained from the intact clostridial neurotoxin by proteolysis, so as to remove or to modify the H_{C} domain.
"Intravesical administration" refers to the injection of a given substance directly into the bladder via a urethral catheter.
"Modified botulinum toxin" means a botulinum toxin that has had at least one of its amino acids deleted, modified, or replaced, as compared to a native botulinum toxin. Additionally, the modified botulinum toxin can be a recombinantly produced neurotoxin, or a derivative or fragment of a recombinantly made neurotoxin. A modified botulinum toxin retains at least one biological activity of the native botulinum toxin, such as, the ability to bind to a botulinum toxin receptor, or the ability to inhibit neurotransmitter release from a neuron. One example of a modified botulinum toxin is a botulinum toxin that has a light chain from one botulinum toxin serotype (such as serotype A), and a heavy chain from a different botulinum toxin serotype (such as serotype B). Another example of a modified botulinum toxin is a botulinum toxin coupled to a neurotransmitter, such as substance P.
"Mutation" means a structural modification of a naturally occurring protein or nucleic acid sequence. For example, in the case of nucleic acid mutations, a mutation can be a deletion, addition or substitution of one or more nucleotides in the DNA sequence. In the case of a protein sequence mutation, the mutation can be a deletion, addition or substitution of one or more amino acids in a protein sequence. For example, a specific amino acid comprising a protein sequence can be substituted for another amino acid, for example, an amino acid selected from a group which includes the amino acids alanine, asparagine, cysteine, aspartic acid, glutamic acid, phenylalanine, glycine, histidine, isoleucine, lysine, leucine, methionine, proline, glutamine, arginine, serine, threonine, valine, tryptophan, tyrosine or any other natural or non-naturally occurring amino acid or chemically modified amino acids. Mutations to a protein sequence can be the result of mutations to DNA sequences that when transcribed, and the resulting mRNA translated, produce the mutated protein sequence. Mutations to a protein sequence can also be created by fusing a peptide sequence containing the desired mutation to a desired protein sequence.
"Patient" means a human or non-human subject receiving medical or veterinary care. Accordingly, as disclosed herein, the compositions and methods can be used in treating any animal, such as, for example, mammals, or the like.
"Permeation-effective amount" refers to an amount effective to substantially increase the permeability of a surface to a therapeutic agent at a therapeutically effective rate. For intravesical bladder delivery, a permeation-effective amount refers to an amount sufficient to substantially increase the permeability of the bladder wall to a clostridial derivative for a desired time interval without irreversibly damaging the bladder wall, after which time the original selective impermeability of the bladder wall may be restored.
"Pharmaceutical composition" means a composition comprising an active pharmaceutical ingredient, such as a botulinum toxin, and at least one additional ingredient, such as a permeabilizing agent. A pharmaceutical composition is therefore a formulation which is suitable for diagnostic or therapeutic administration to a subject, such as a human patient. The pharmaceutical composition can be, for example, in a lyophilized or vacuum dried condition, a solution formed after reconstitution of the lyophilized or vacuum dried pharmaceutical composition, or as a solution or solid which does not require reconstitution.
   The constituent ingredients of a pharmaceutical composition can be included in a single composition (that is, all the constituent ingredients, except for any required reconstitution fluid, are present at the time of initial compounding of the pharmaceutical composition) or as a two-component system, for example a vacuum-dried composition reconstituted with a reconstitution vehicle which can, for example, contain an ingredient not present in the initial compounding of the pharmaceutical composition. A two-component system can provide several benefits, including that of allowing incorporation of ingredients which are not sufficiently compatible for long-term shelf storage with the first component of the two component system. For example, the reconstitution vehicle may include a preservative which provides sufficient protection against microbial growth for the use period, for example one-week of refrigerated storage, but is not present during the two-year freezer storage period during which time it might degrade the toxin. Other ingredients, which may not be compatible with a botulinum toxin or other ingredients for long periods of time, can be incorporated in this manner; that is, added in a second vehicle (e.g. in the reconstitution vehicle) at the approximate time of use. A pharmaceutical composition can also include preservative agents such as benzyl alcohol, benzoic acid, phenol, parabens and sorbic acid. Pharmaceutical compositions can include, for example, excipients, such as surface active agents; dispersing agents; inert diluents; granulating and disintegrating agents; binding agents; lubricating agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; antioxidants; stabilizing agents; and pharmaceutically acceptable polymeric or hydrophobic materials and other ingredients known in the art and described, for example in Genaro, ed., 1985, Remington's Pharmaceutical Sciences, Mack Publishing Co., Easton, Pa..
"Recombinant modified toxin" means a recombinant toxin that shares some or most of the domains with a Botulinum toxin but may or may not target the same cells as native Botulinum neurotoxin.
"Stabilizing", "stabilizes", or "stabilization" means the retention of at least about 20% of the biological activity of an active pharmaceutical ingredient ("API") that has been reconstituted, when compared to the API prior to reconstitution. For example, upon (1) preparation of serial dilutions from a bulk or stock solution, or (2) upon reconstitution of a lyophilized, or vacuum dried botulinum toxin containing pharmaceutical composition which has been stored at or below about -2°C for between six months and four years, or (3) for an aqueous solution botulinum toxin containing pharmaceutical composition which has been stored at between about 2°C and about 8°C for from six months to four years, the botulinum toxin present in the reconstituted or aqueous solution pharmaceutical composition has (in the presence of a compound which is stabilizing, stabilizes or which provides stabilization to the API) greater than about 20% and up to about 100% of the potency or toxicity that the biologically active botulinum toxin had prior to being incorporated into the pharmaceutical composition.
"Stabilizing agent", "stabilization agent" or "stabilizer" means a substance that acts to stabilize an API such that the potency of the pharmaceutical composition is increased relative to an unstabilized composition.
"Stabilizers" can include excipients, and can include protein and non-protein molecules.
"Substantially free" means present at a level of less than one percent by weight of the pharmaceutical composition.
"Therapeutic formulation" means a formulation can be used to treat and thereby alleviate a disorder or a disease, such as, for example, a disorder or a disease characterized by hyperactivity (*i.e.* spasticity) of a peripheral muscle.
"Therapeutically effective amount" refers to an amount sufficient to achieve a desired therapeutic effect.
"Treating" means to alleviate (or to eliminate) at least one symptom of a condition or disorder, such as, neurogenic bladder dysfunction either temporarily or permanently.
"Variant" means a wild-type botulinum toxin serotype A, B, C, D, E, F or G, that has been modified by the replacement, modification, addition or deletion of at least one amino acid relative to wild-type botulinum toxin, which is recognized by a target cell, internalized by the target cell, and catalytically cleaves a SNARE (SNAP (Soluble NSF Attachment Protein) Receptor) protein in the target cell.
   An example of a variant neurotoxin component can comprise a variant light chain of a botulinum toxin having one or more amino acids substituted, modified, deleted and/or added. This variant light chain may have the same or better ability to prevent exocytosis, for example, the release of neurotransmitter vesicles. Additionally, the biological effect of a variant may be decreased compared to the parent chemical entity. For example, a variant light chain of a botulinum toxin type A having an amino acid sequence removed may have a shorter biological persistence than that of the parent (or native) botulinum toxin type A light chain.
"Vehicle" or "reconstitution vehicle" means a liquid composition that can be used to reconstitute a solid botulinum formulation into a liquid botulinum pharmaceutical composition.
"Wild type neuronal binding moiety" means that portion of a neurotoxin which is native to the neurotoxin and which exhibits a specific binding affinity for a receptor on a neuron. Thus, wild type or native neuronal binding moiety excludes a binding moiety with is not native to the neurotoxin.

### Pharmaceutical compositions

Aspects of the present disclosure provide, in part, a pharmaceutical composition suitable for intravesical bladder delivery, comprising a clostridial derivative and at least one permeabilizing agent as disclosed herein.

### Clostridial derivative:

A clostridical derivative as defined herein is a native or modified botulinum toxin as defined herein. In one embodiment, the clostridial derivative is a botulinum toxin type A. In some embodiments, the clostridial derivative is a botulinum type B, C₁, D, E or F.

In some embodiments, the present pharmaceutical composition comprises a therapeutically effective amount of the clostridial derivative. A therapeutically effective amount refers to the total amount of the clostridial derivative administered to an individual in one setting. As such, an effective amount of a Clostridial derivative does not refer to the amount administered per site. For example, an effective amount of a botulinum toxin, administered to an individual may be 10U, whereas the amount of toxin administered per site may be 2U, *i.e*., 2 U at five different sites. In some embodiments, the therapeutically effective amount of the botulinum toxin ranges from 10 U to 1000 U, more preferably from about 50 U to about 500 U.

With reference to a combination therapy comprising a Clostridial toxin and a TEM, an effective amount of a Clostridial toxin is one where in combination with a TEM the amount of the Clostridial toxin achieves the desired therapeutic effect, but such an amount administered on its own would be ineffective. For example, typically about 75-125 U of BOTOX® (Allergan, Inc., Irvine, CA), a BoNT/A, is administered by intramuscular injection per muscle undergoing dystonic spasms in order to treat cervical dystonia. In combination therapy, a suboptimal effective amount of BoNT/A would be administered to treat cervical dystonia when such toxin is used in a combined therapy with a TEM.

### Permeabilizing agents

The at least one permeabilizing agent comprises a nonionic surfactant and a mucoadhesive, which is a cationic polymer.

In some embodiments, the permeabilizing agent selectively binds to the botulinum toxin, to form a complex. In alternative embodiments, the permeabilizing agent does not bind to the botulinum toxin. In alternative embodiments, the permeabilizing agent interacts with the botulinum toxin through Van der Waal interactions.

In certain embodiments, the permeabilizing agent can comprise polyethylene glycol (PEG) or polyethylene oxide (PEO). The PEG can comprise, for example, PEG with a molecular mass ranging from about 200 grams per mole (g/m) to about 20,000 grams per mole (g/m). In one embodiment, the permeabilizing agent comprises Polyethylene glycol 3350.

In certain embodiments, the permeabilizing agent can comprise a poloxamer. The poloxamer can comprise, for example, P80, P124, P188, P237, P338, and P407, their analogs, derivatives or combinations thereof. In certain embodiments the permeabilizing agent can comprise a povidone (PVP). The PVP can comprise, for example, PVP polymers, and analogs or derivatives.

The permeabilizing agent comprises a non-ionic surfactant. Examples of nonionic surfactants suitable for the present formulation include but are not limited to alkyl aryl polyethers and analogs, derivatives thereof (e.g. TX-100, analogs or derivatives), poloxamers, polyoxyethylene ethers (e.g. Brij families), Tween, Big CHAPS, Deoxy Big CHAPS, Sorbitan monooleate (SPAN) 20, 40, 60, Cremophor EL, Alpha-Tocopherol TPGS, Polyoxyl stearate 40, analogs and derivatives, or combinations thereof.

In some embodiments, the permeabilizing agent comprises an alkyl aryl polyether, analogs or derivatives. In one embodiment, the permeabilizing agent comprises octyl phenol ethoxylate, analogs or derivatives. Octyl phenol ethoxylate is also known as polyoxyethylene octyl phenyl ether, 4-octylphenol polyethoxylate, Mono 30, TX-100, t-octylphenoxypolyehtoxyethanol, octoxynol-9, or the more commonly known tradename of Triton™ X-100. In alternative embodiments, the permeabilizing agent comprises Nonoxynol 9, analogs, or derivatives.

The permeabilizing agent further comprises a cationic polymer The cationic polymer is a mucoadhesive. In some embodiments, the cationic polymer includes chitosan.

In some embodiments, the at least one permeabilizing agent comprises chitosan and TX-100, TX-100 analogs or TX-100 derivatives. In some embodiments, the present pharmaceutical composition comprises a botulinum toxin, chitosan, and nonoxynol-9, nonoxynol-9 analogs or nonoxynol-9 derviatives.

In certain embodiments, the permeabilizing agent can comprise chelators, including but not limited to EDTA, EGTA (ethylene glycol tetraacetic acid), cyclohexanediamine tetraacetate (CDTA), hydroxyethylethylenediamine triacetate (HEDTA), diethylenetriamine pentaacetate (DTPA), 1 ,2-diaminocyclohexane tetraacetate, and hexametaphosphate. These agents preferably are employed as salts, typically sodium salts such as disodium EDTA, trisodium HEDTA, sodium hexametaphosphate, or any combinations thereof.

Thus, in one aspect, the present pharmaceutical composition comprises a clostridial derivative, and at least one permeabilizing agent, wherein the pharmaceutical formulation is suitable for intravesical bladder delivery, wherein the clostridial derivative comprises a botulinum toxin and the permeabilizing agent comprises a mucoadhesive and a non-ionic surfactant. In some embodiments, the mucoadhesive includes chitosanIn one embodiment, the present pharmaceutical composition comprises a botulinum toxin type A, chitosan and TX-100, TX-100 analogs or TX-100 derivatives. In some embodiments, the non-ionic surfactant comprises nonoxynol-9, nonoxynol-9 analogs or nonoxynol-9 derviatives.

The at least one permeabilizing agent is present in a permeation-effective amount.A permeation effective amount refers to an amount effective to substantially increase the permeability of the bladder wall surface with limited damage to the bladder integrity. In one embodiment, the permeation-effective amount refers to an amount effective to allow permeation of a therapeutically effective amount of a botulinum neurotoxin through the bladder wall at a therapeutically effective rate. In one embodiment, the permeation effective amount refers to an amount effective to substantially increase the permeability of the bladder wall surface for a desired time interval to a therapeutically effective amount of the toxin at a therapeutically effective rate without irreversibly damaging the bladder wall., The increased permeability is reversible after a desired time interval, after which the bladder wall may fully or partially restore its original impermeability or selective permeability. In some embodiments, the bladder wall restores its original impermeability or selective permeability after a time interval ranging from about 1 hour to about 24 hours after intravesical instillation. In some embodiments, the time interval ranges from about 3 hours to about 18 hours. In some embodiments, the time interval ranges from about 4 hours to about 12 hours. The recovery rate whereby the bladder wall restores its original selective permeability or impermeability can be influenced by the characteristics of the permeabilizing agent selected, the amount, the characteristics of the permeation surface, the exposure time and the environment surrounding the permeation surface. In one embodiment, the bladder integrity following administration of the present pharmaceutical composition can be evaluated by the extent of immune response, such as the presence of specific immune cells.

The permeation-effective amount varies depending on several factors, including but not limited to the characteristics of the clostridial derivative, the characteristics of the permeation surface (e.g. the bladder wall), the type of permeabilizing agent, and the environment surrounding the permeation surface.

In some embodiments, the permeation-effective amount of the permeabilizing agent ranges from about 0.005% to about 10% (w/v), more preferably from about 0.025% to about 5% (w/v), and most preferably from about 0.05% to about 0.5% (w/v). In some embodiments, the present pharmaceutical formulation comprises a permeation-effective amount of Triton™ X-100, Triton™ X-100 analogs or derivatives from 0.005% to about 10% (w/v), more preferably from about 0.025% to about 5% (w/v), and most preferably from about 0.1% to about 0.5% (w/v). In one specific embodiment, the permeative effective amount of Triton™ X-100 is about 0.1% (w/v).

The permeabilizing agent is used in combination with a mucoadhesive. The mucoadhesive is a cationic polymer. In some embodiments, the mucoadhesive includes chitosan. In some embodiments, the permeation-effective amount of the mucoadhesive ranges from about 0.005% to 10% (w/v), more preferably from about 0.02% to about 5% (w/v), and most preferably from about 0.05% to about 2% (w/v). In one embodiment, the permeation-effective amount of chitosan is about 1% (w/v). In some embodiments, the formulation comprises: (1) a botulinum toxin, (2) Triton™ X-100, Triton™ X-100 analog, Triton™X-100 derivatives or mixtures thereof; and (3) chitosan, or mixtures thereof. In some embodiments, the formulation comprises: (1) a botulinum toxin type A, (2) Triton™X-100, Triton™X-100 analogs, Triton™X-100 derivatives, or mixtures thereof; and (3) chitosan, or mixtures thereof. In some embodiments, the formulation comprises about 20 units to about 300 units of botulinum toxin type A, about 0.5% to about 2% (w/v) chitosan; or mixtures thereof, and about 0.05% to about 1% (w/v) Triton™X-100, analogs, derivatives or mixtures thereof. In one embodiment, the formulation comprises from about 100 or 200 units of botulinum toxin type A, about 1% chitosan and about 0.1% (w/v) Triton™ X-100.

In some embodiments, the permeabilizing agent comprises Nonoxynol 9. In some embodiments, the permeation-effective amount of Nonoxynol 9 ranges from about 0.005% to about 10% (w/v), more preferably from about 0.025% to about 5% (w/v), and most preferably from about 0.05% to about 0.5% (w/v). In one specific embodiment, the present pharmaceutical formulation comprises a permeation-effective amount of Nonoxynol 9 of about 0.1% (w/v). In one specific embodiment, the present pharmaceutical formulation comprises a permeation-effective amount of Nonoxynol 9 of about 0.5% (w/v). In some embodiments, nonoxynol 9 is used in combination with a mucoadhesive. In some embodiments, the mucoadhesive includes chitosan. In some embodiments, the permeation-effective amount of the mucoadhesive ranges from about 0. 005% to 10% (w/v), more preferably from about 0.02% to about 5% (w/v), and most preferably from about 0.1% to about 2% (w/v). In some embodiments, the permeation-effective amount of chitosan range from about 0.005% to 10% (w/v), more preferably from about 0.02% to about 5% (w/v), and most preferably from about 0.1% to about 2% (w/v). In one embodiment, the permeation-effective amount of chitosan is about 1% (w/v).

Thus, aspects of the present disclosure provide a pharmaceutical composition comprising a clostridial derivative and one or more permeabilizing agents as disclosed above, wherein the pharmaceutical composition is suitable for intravesical bladder delivery and wherein the one or more permeabilizing agent is present in a permeation-effective amount, as disclosed herein. Embodiments of the present composition include therapeutic agents and/or excipients that will either cause or enhance the pharmacological effects of thebotulinum toxins.

Excipients can also be added to increase the stability of the formulation, increase the action of permeablizing agents (example EDTA or other chelating agents) or to increase the retention of the formulation through increased viscolastic properties that will occur immediately (example: carboxymethyl cellulose (CMC), alginate) or upon change in temperature (example: poloxamer 407), pH (Carbopol P-934, P940) and/or ionic (example: Gelrite gellan gum, alginate) environments.

Excipients can also be added to modulate the tonicity and/or pH of urine and skin to increase delivery, stability, bioavailability and/or therapeutic activity of formulations.

In another aspect, the present disclosure provides a method for making a pharmaceutical formulation suitable for intravesical bladder administration, the method comprising providing a solution comprising at least one permeabilizing agent as disclosed herein, adding the solution to a composition comprising a clostridial derivative as disclosed herein. In some embodiments, the method comprises adding from about 50 ml to about 100ml of the solution to the composition comprising the botulinum toxin. In one embodiment, the method comprises adding about 50ml to about 100 ml of an aqueous solution comprising about 1% (w/v) chitosan and about 0.1% (w/v) Triton™X-100, analogs or derivatives, to a botulinum toxin type A. In one embodiment, the method comprises adding a 50ml aqueous solution comprising about 1% (w/v) chitosan and about 0.1% (w/v) Triton™X-100 to a vial containing about 100 Units or 200 Units of a lyophilized botulinum toxin type A; and mixing gently to rehydrate the lyophilized botulinum toxin type A.

In some embodiments, excipients can also be added to act as carriers of the clostridial derivative. In one embodiment, poly-L-lysine is added as a carrier.

A composition disclosed herein is generally administered as a pharmaceutical acceptable composition. As used herein, the term "pharmaceutically acceptable" means any molecular entity or composition that does not produce an adverse, allergic or other untoward or unwanted reaction when administered to an individual. As used herein, the term "pharmaceutically acceptable composition" is synonymous with "pharmaceutical composition" and means a therapeutically effective concentration of an active ingredient, such as, the botulinum toxin. A pharmaceutical composition disclosed herein is useful for medical and veterinary applications. A pharmaceutical composition may be administered to an individual alone, or in combination with other supplementary active ingredients, agents, drugs or hormones. The pharmaceutical compositions may be manufactured using any of a variety of processes, including, without limitation, conventional mixing, dissolving, granulating, dragee-making, levigating, emulsifying, encapsulating, entrapping, and lyophilizing. The pharmaceutical composition can take any of a variety of forms including, without limitation, a sterile solution, suspension, emulsion, lyophilizate, tablet, pill, pellet, capsule, powder, syrup, elixir or any other dosage form suitable for administration.

The present pharmaceutical composition may optionally include a pharmaceutically acceptable carrier that facilitates processing of an active ingredient into pharmaceutically acceptable compositions. As used herein, the term "pharmacologically acceptable carrier" is synonymous with "pharmacological carrier" and means any carrier that has substantially no long term or permanent detrimental effect when administered and encompasses terms such as "pharmacologically acceptable vehicle, stabilizer, diluent, additive, auxiliary or excipient." Such a carrier generally is mixed with an active compound, or permitted to dilute or enclose the active compound and can be a solid, semi-solid, or liquid agent. It is understood that the active ingredients can be soluble or can be delivered as a suspension in the desired carrier or diluent. Any of a variety of pharmaceutically acceptable carriers can be used including, without limitation, aqueous media such as, e.g., water, saline, glycine, hyaluronic acid; solid carriers such as, e.g., mannitol, lactose, starch, magnesium stearate, sodium saccharin, talcum, cellulose, glucose, sucrose, magnesium carbonate; solvents; dispersion media; coatings; antibacterial and antifungal agents; isotonic and absorption delaying agents; or any other inactive ingredient. Selection of a pharmacologically acceptable carrier can depend on the mode of administration. Except insofar as any pharmacologically acceptable carrier is incompatible with the active ingredient, its use in pharmaceutically acceptable compositions is contemplated. Non-limiting examples of specific uses of such pharmaceutical carriers can be found in PHARMACEUTICAL DOSAGE FORMS AND DRUG DELIVERY SYSTEMS (Howard C. Ansel et al., eds., Lippincott Williams & Wilkins Publishers, 7th ed. 1999); REMINGTON: THE SCIENCE AND PRACTICE OF PHARMACY (Alfonso R. Gennaro ed., Lippincott, Williams & Wilkins, 20th ed. 2000); GOODMAN & GILMAN'S THE PHARMACOLOGICAL BASIS OF THERAPEUTICS (Joel G. Hardman et al., eds., McGraw-Hill Professional, 10th ed. 2001); and HANDBOOK OF PHARMACEUTICAL EXCIPIENTS (Raymond C. Rowe et al., APhA Publications, 4th edition 2003). These protocols are routine procedures and any modifications are well within the scope of one skilled in the art and from the teaching herein.

A pharmaceutical composition disclosed herein can optionally include other pharmaceutically acceptable components (or pharmaceutical components), includingbuffers, preservatives, tonicity adjusters, salts, antioxidants, osmolality adjusting agents, physiological substances, pharmacological substances, bulking agents, emulsifying agents, wetting agents, sweetening or flavoring agents. Various buffers and means for adjusting pH can be used to prepare a pharmaceutical composition disclosed herein, provided that the resulting preparation is pharmaceutically acceptable. Such buffers includeacetate buffers, citrate buffers, phosphate buffers, neutral buffered saline, phosphate buffered saline and borate buffers. It is understood that acids or bases can be used to adjust the pH of a composition as needed. Pharmaceutically acceptable antioxidants includesodium metabisulfite, sodium thiosulfate, acetylcysteine, butylated hydroxyanisole and butylated hydroxytoluene. Useful preservatives includebenzalkonium chloride, chlorobutanol, thimerosal, phenylmercuric acetate, phenylmercuric nitrate, a stabilized oxy chloro composition and chelants, such as, *e.g*., DTPA or DTPA-bisamide, calcium DTPA, and CaNaDTPA-bisamide. Tonicity adjustors useful in a pharmaceutical composition include, without limitation, salts such as, e.g., sodium chloride, potassium chloride, mannitol or glycerin and other pharmaceutically acceptable tonicity adjustor. The pharmaceutical composition may be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. It is understood that these and other substances known in the art of pharmacology can be included in a pharmaceutical composition. Exemplary pharmaceutical composition comprising a Clostridial toxin and a TEM are described in Hunt, et al., Animal Protein-Free Pharmaceutical Compositions, US Serial No. 12/331,816; and Dasari, et al., Clostridial Toxin Pharmaceutical Compositions, WO/2010/090677,.

The choice of suitable pharmaceutically acceptable carriers will depend on the exact nature of the particular formulation desired, e.g., whether the present composition is to be formulated into a liquid solution, a lyophilized powder to be reconstituted upon use, a suspension solution, nanoparticles, liposomes or microemulsions.

The choice of a suitable pharmaceutically acceptable carrier will also depend on the route of administration. Preferably, the carrier is formulated to be suitable for a chosen route of administration. Administration modes of the present pharmaceutical formulation include but are not limited to injection, needle-free injections (e.g. Bioject, JetTouch), electromotive, transdermal delivery or intravesicle instillation. In one specific embodiment, the formulation comprises a pharmaceutical acceptable carrier for bladder instillation. In one embodiment, the pharmaceutical carrier comprises poly lysine.

Compositions for use of treating neurogenic bladder dysfunctionAspects of the present disclosure provide, in part, a a pharmaceutical composition comprising a clostridial derivative and at least one permeabilizing agent as described above for use in treating a medical condition, wherein administration of the present pharmaceutical composition prevents or reduces a symptom associated with the medical condition being treated. In one embodiment the administration is by intravesical delivery.

### Treatment of urological disorders

In some embodiments, the medical disorder comprises urological bladder diseases and conditions, including overactive bladder (OAB), cystitis, bladder cancer, neurogenic detrusor overactivity (NDO). In an embodiment, the present disclosure also provides compositions for use for treating a patient suffering from overactive bladder (OAB), such as, for example, that due to a neurologic condition (NOAB), or idiopathic OAB (IOAB).

Neurogenic bladder dysfunction is a dysfunction that results from interference with the normal nerve pathways associated with urination. One type of neurogenic bladder dysfunction is overactive (spastic or hyper-reflexive) bladder. An overactive neurogenic bladder is characterized by uncontrolled, frequent expulsion of urine from the bladder. There may be reduced bladder capacity and incomplete emptying of urine. Spastic bladder may be caused by an inability of the detrusor muscle of the bladder to inhibit emptying contractions until a reasonable amount of urine has accumulated. Often, a strong urge to void is experienced when only a small amount of urine is in the bladder. The patient may report symptoms of urgency, frequency, nocturia, and incontinence. Another type of neurogenic bladder dysfunction is characterized by difficulty in relaxing the urinary sphincter muscle. The sphincter may be spastic. This causes difficulty in emptying the bladder, which can lead to urinary retention and urinary tract infections. In another type of neurogenic bladder dysfunction, both the detrusor muscle and the urinary sphincter simultanously contract resulting in urinary retention. A dysfunction associated with simultaneous contraction of both the detrusor and the urinary sphincter is called detrusor-external sphincter dyssynergia (DESD). U.S. Pat. Nos. 7,449,192; 8,062,807 and 7,968,104disclose compositions for use for treating a patient with a neurogenic bladder dysfunction by injecting a therapeutically effective amount of a botulinum toxin, into the bladder wall of the patient.

Interstitial cystitis (IC) is an incurable, chronic, debilitating disease of the urinary bladder that is characterized by bladder pain, chronic pelvic pain, irritative voiding symptoms and sterile urine. In IC, the bladder wall typically shows inflammatory infiltration with mucosal ulceration and scarring which causes smooth muscle contraction, diminished urinary capacity, hematuria and frequent, painful urination.

In some embodiments, pharmaceutical formulations of the present disclosure can be administered to the bladder or its vicinity, e.g. the detrusor, wherein the administration of the formulation reduces the urge incontinence associated with overactive bladder. In certain embodiments, the dosage can range from about 10 Units to about 200U per treatment. In some embodiments, the present pharmaceutical formulations can be administred by intravesical bladder delivery.

Thus, aspects of the present disclosure further provide a composition for use for treating a bladder condition in a patient in need thereof, comprising: providing a pharmaceutical composition as disclosed herein, comprising a clostridial derivative which is a botulinum toxin, and at least one permeabilizing agent comprising a non-ionic surfactant and a mucoadhesive, which is a cationic polymer, wherein the permeabilizing agent is present in an amount effective to substantially enhance the permeability of the bladder wall to the clostridial derivative, at a therapeutically effective rate, and without irreversibly damaging the bladder wall; and instilling the pharmaceutically composition to the bladder via a catheter; thereby treating the bladder conditions. In one embodiment, the composition for use further comprises pre-treating the bladder wall with the pharmaceutical composition prior to the step of instilling.

In some embodiments, the composition for use for treating a bladder condition in a patient comprising providing a solution comprising a permeabilizing agent; adding the solution to a clostridial derivative to form a therapeutic formulation, instilling the therapeutic formulation through a catheter into a patient's bladder. The clostridial derivative is a botulinum toxin. In one embodiment, the clostridial derivative is a botulinum toxin type A. The permeabilizing agent comprises a non-ionic surfactant and a mucoadhesive, which is a cationic polymer. In some embodiments, the mucoadhesive comprises chitosan.

In another aspect, the present disclosure provides a method for alleviating the symptoms of Interstitial cystitis (IC) in a patient, the method comprising: providing a pharmaceutical composition for use, comprising a clostridial derivative and at least one permeabilizing agent, wherein the permeabilizing agent is present in an amount effective to substantically enhance the permeability of the bladder wall to the clostridial derivative at a therapeutically effective rate, and without irreversibly damaging the bladder wall; and instilling the pharmaceutically composion to the bladder via a catheter; thereby alleviating the symptoms of the IC.

In another aspect, the present formulation maximizes the bioavailability of a clostridial derivative by preventing or minimizing the adsorption of the clostridial derivative to catheters, deliver device surfaces (syringe, patch, microneedle, engineered injector (Bioject, etc.), tubing and containers.

In another aspect, the present formulation maximizes the bioavailability of the clostridial derivative by enhancing the retention of the clostridial derivative to the skin or inner bladder wall surface, through mucoadhesive interactions.

### EXAMPLES

The following examples illustrate embodiments and aspects of the present invention.

### Example 1

The purpose of this study was to evaluate the effect of botulinum toxin type A, known as BOTOX®, in one of eight vehicle formulations administered by instillation into the urinary bladder of female Sprague Dawley rats. The effect was examined eight days after administration wherein efficacy and tolerability were evaluated by immunohistochemistry (IHC) and histopathology, respectively.

Positive controls: Four rats were administered 10 units of BOTOX® in saline by injection into the detrusor muscle.

Negative controls: Formulations containing the vehicle only (as shown in Table 1 without addition of BOTOX®).

Cleaved SNAP 25 was used as a biomarker of BOTOX® activity at synaptic terminals and a potential indicator of functionality of the method of delivery. In this study it was used to confirm the successful movement of BOTOX® across the urothelium. Synaptophysin expression was used to identify synaptic terminals and to ensure specificity of cleaved SNAP 25 localization. Histopathology was done to assess impact of the formulation on the bladder tissue.

Female rats weighing between 150-200 grams were administered 0.5ml vehicle formulation or vehicle formulation + 30 U Botox® according to Table 1, whereby only the vehicles described in DP Study Numbers 5 and 8 are according to the present claims:

**Table 1:**

| UR13002RS Study Number | DP Study Number | Vehicle Formulation |
|---|---|---|
| 1 | DP13104 | 0.1% Triton |
| 2 | DP13112 | 0.2% Triton |
| 3 | DP13117 | 0.05% Triton |
| 4 | DP13121 | 0.1% Nonoxynol-9^{a} |
| 5 | DP13129 | 1% Chitosan + 0.1% Triton |
| 6 | DP13130 | 0.25% HPMC +0.1% Triton |
| 7 | DP13139 | 0.1% Tyloxapol |
| 8 | DP13145 | 1% Chitosan + 0.1% Nonoxynol-9 |

| | | |
|---|---|---|
| ^{a}= only 5 vehicle + BOTOX treated bladders submitted for evaluation. | | |

Intravesical instillation of the formulation was carried out as follows: rats were anesthetized with isoflurane and the bladder emptied by way of finger tip pressure applied on the lower abdomen. While under anesthesia, a catheter was introduced into the urinary bladder via the urethra. Subsequently, the formulation was slowly administered over a course of two minutes into the urinary bladder at a dose volume of 0.5ml + 0.1ml (to accommodate for the dead space created by the catheter). The formulation was allowed to dwell in the bladder for 60minutes before anesthesia recovery. The rats were euthanized one week after instillation.

Urinary bladders were collected, fixed in 10% formalin and processed using standard histological techniques. Additional sections were prepared and processed for fluorescent immunohistochemistry for cleaved SNAP 25 and synaptophysin.

**Immunofluorescence:** One tissue block per animal was cryostat-sectioned (14 µm-thick) in the longitudinal plane and slide-mounted. Three slides were prepared from each block, with three sections on each slide, approximately 140 µm apart. Two slides were processed for immunofluorescence. Tissue sections were first blocked for nonspecific signal in blocking buffer (1X PBS + 0.1% TX-100 + 10% Normal Donkey Serum) and then incubated with combinations of primary antibodies, including anti-cleaved SNAP25 and anti-synaptophysin, at the desired concentration in blocking buffer, overnight at 4°C. Following washes, sections were incubated with secondary antibodies (Jackson ImmunoResearch) for 2 hr at 4°C and then washed again. Slide-mounted sections were coversliped using Fluoromount-G with 1.5 µg/ml DAPI. The third slide was stained with Hematoxylin & Eosin (H&E) for anatomical assessment.

**Data analysis and quantitation:** Images were analyzed and captured on either a Leica DMLB brightfield microscope, a Nikon E800 fluorescent widefield microscope or a Zeiss LSM-710 confocal microscope using Image-Pro® (MediaCybernectics), Metamorph® (Molecular Devices) or ZEN (Carl Zeiss) software. Imaris® (Bitplane) software was used for high-resolution, 3D qualitative analysis to establish the spatial relationships of nerve fibers. Nerve fiber-types were identified on the basis of their morphology and neurochemistry. For the semi-quantitative analysis, for each slide, all 3 sections were carefully observed under a microscope and a score (from 0 to 4) was given for each animal on either the extent of SNAP25₁₉₇ staining (as shown in Figs. 3 and 4) or on the integrity of the bladder anatomy (H&E) (as shown in Figs. 5A-D). An average score was calculated for each treatment/formulation and the results are partly presented in Table 2 and Fig. 6.

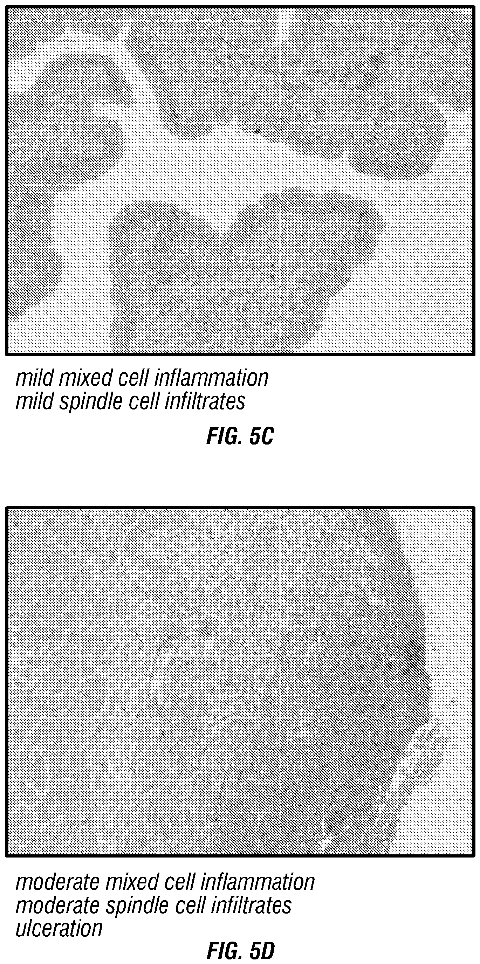

The formulations were evaluated based on the following:
1. Histological changes in the bladder wall; wherein a diagnosis of "spindle cell infiltrate" was made for bladders in which an increased number of fusiform cells of indeterminant origin were found infiltrating the lamina propria (Fig. 5); as a reactive change, these were considered undesirable as a potential precursor to fibrosis; and
2. Cleaved SNAP 25 score.

Summary of results: The results are summarized in Table whereby only Rat # 35-39, 41, 59-61, 63, 64, and 66 received treatments as claimed:

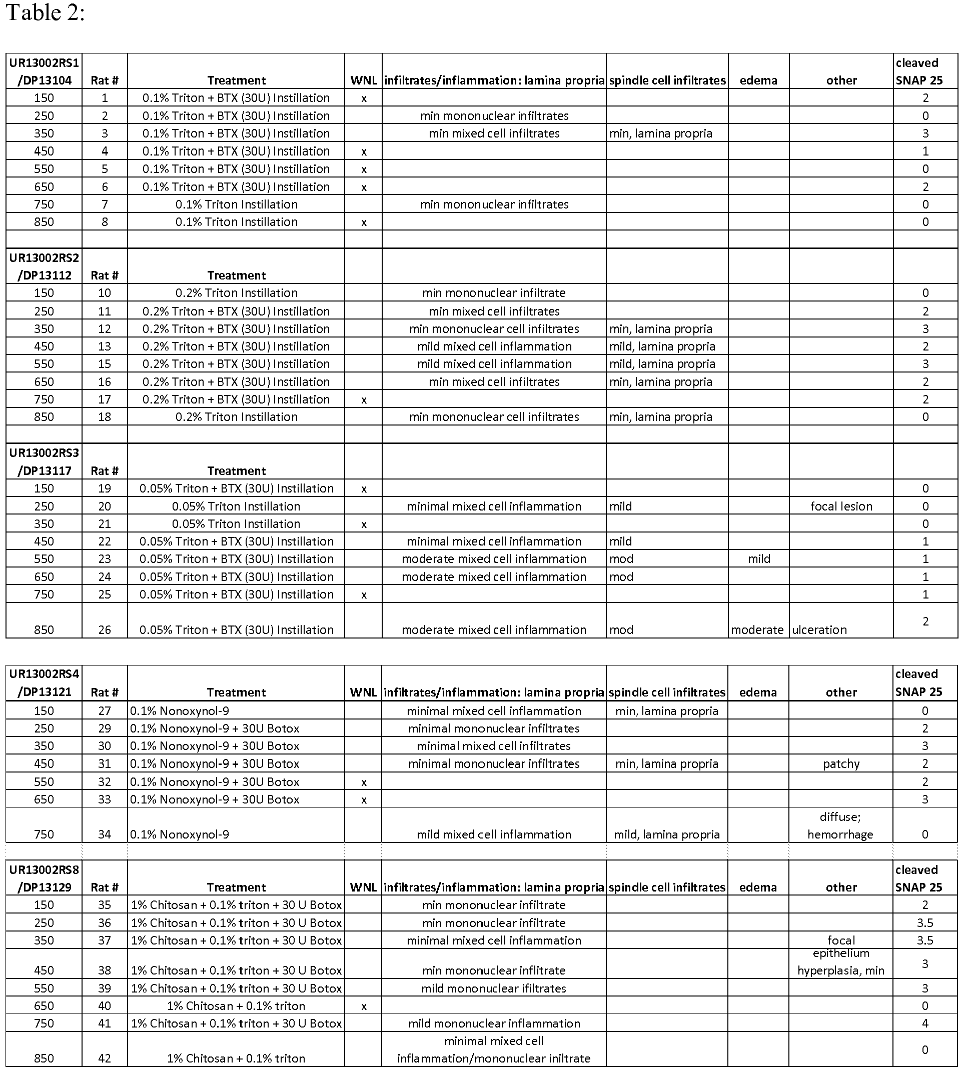

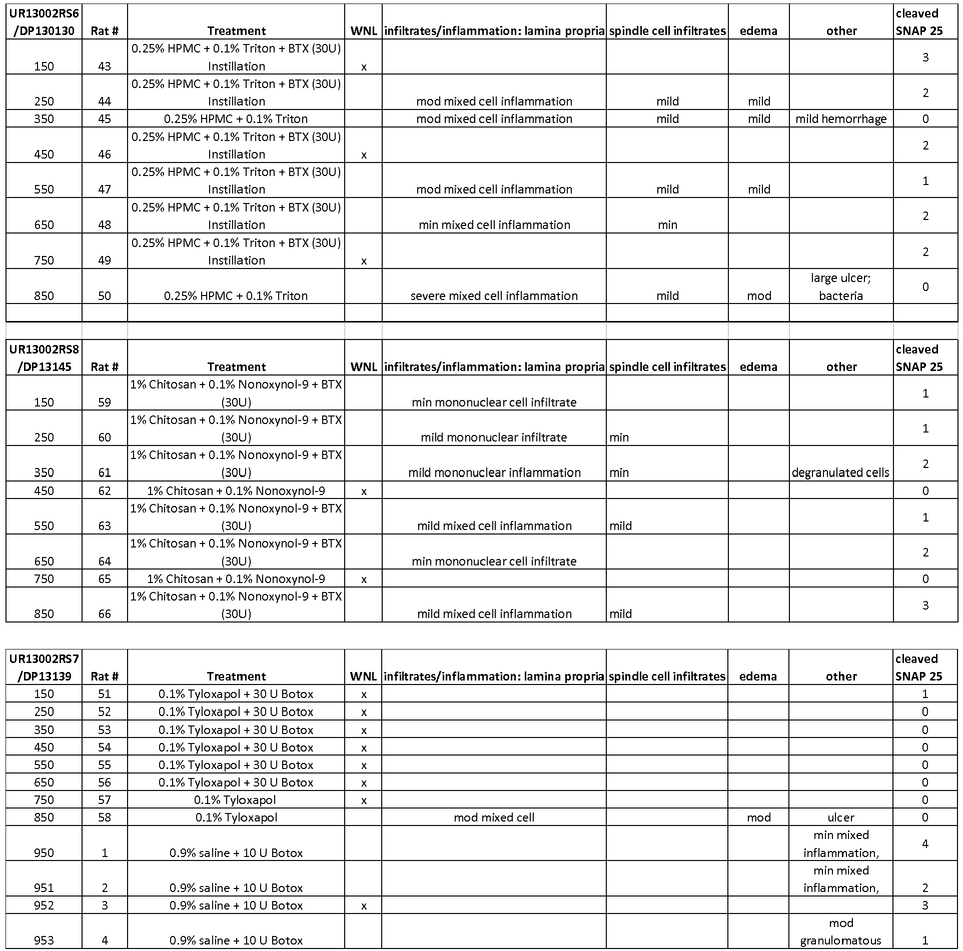

Test formulations: Positive immunohistory scores were observed in all samples post intravesical instillation of BOTOX®, except for the formulations of 0.1% (w/v) Tyloxapol. Figure 6 shows the IHC scores of some exemplary formulations.

### Example 2

### Treatment of overactive bladder

This example describes treatment of patients with hyper reflexive bladder due to neurogenic or idiopathic bladder dysfunction.

Several patients with hyper reflexive bladders symptoms (bladder infection, incontinence, and urge incontinence) due to neurogenic or idiopathic bladder dysfunction are treated by bladder instillation. A pharmaceutical composition comprising about 100 Units of BOTOX®, 0.1% (w/v) Triton™ X-100 and 1% (w/v) chitosan . The pharmaceutical composition is instilled to the bladder of the patients while under light sedation. A significant increase in mean maximum bladder capacity and a significant decrease in mean maximum detrusor voiding pressure are observed 7 days post treatment.

### Reference Example 3: Triton X-100 induced increased permeability of the bladder wall was reversible

Human bladder uroepithelial cells (CELLnTEC Cat # HBEP.05) were plated at a concentration of approximately 150,000 viable cells per well on polycarbonate membrane inserts. The inserts were placed inside the wells of 24 well tissue culture plates. CELLnTEC CnT-58 media (growth media) was then added to the wells and the cells were incubated for 2 days at 37 °C (5% CO₂) until cells were confluent. At the end of incubation, growth media was removed and replaced with CELLnTEC CnT-21 media (differentiation media). Cells were then allowed to differentiate for 7 days after which a 2 to 3 cell human uroepithelial layer was established. Cells were treated for one hour with the following vehicles: 0.9% saline, 0.1% Triton X-100 in 0.9% saline and 0.5% Triton X-100 in saline. Each vehicle was tested in triplicate. Cells were treated by applying 0.1 mL volumes of each vehicle to the surfaces of the membrane inserts in which cells were grown on. After exposure vehicle solutions were removed from the inserts and differentiation media was added to each insert. Cells were then incubated and allowed to recover for either 0, 24, or 48 hours. Note that 0.9% saline (negative control) was only tested at 0 hours. Gelatin permeability assays were then performed. Gelatin permeability assays were performed by removing media from each insert followed by adding 0.1 mL volumes of 0.1 mg/ mL Oregon Green 488 labeled gelatin, formulated in saline, to each insert. Inserts were placed into wells containing 0.8 mL volumes of Earl's Balanced Salt Solution. After 1 hour exposure, flow through was collected and measured for fluorescence using an Envision instrument. Twenty-four hour and forty-eight hour test results were then compared to 0 hour data to determine if decreases in gelatin permeability had occurred.

It was found that that the amount of gelatin that diffused through the in vitro urothelium treated with 0.1% Triton X-100 was lower after 24 and 48 hours recovery. These results suggest recovery of in vitro human urothelial permeability after treatment with 0.1% Triton X-100.

## Claims

1. A pharmaceutical composition comprising a therapeutically effective amount of a clostridial derivative and at least one permeabilizing agent,
wherein the clostridial derivative is a botulinum toxin, and wherein the at least one permeabilizing agent comprises a non-ionic surfactant and a mucoadhesive, which is a cationic polymer, and
wherein the at least one permeabilizing agent is present in an amount effective to substantially and reversibly increase the permeability of the bladder wall to the clostridial derivative.

2. The pharmaceutical composition of claim 1, wherein the surfactant comprises an alkyl aryl polyether.

3. The pharmaceutical composition of claim 1, wherein the mucoadhesive comprises chitosan.

4. The pharmaceutical composition of claim 1, wherein the surfactant is present in an amount ranging from 0. 01% to 0.5% (w/v).

5. The pharmaceutical composition of claim 2, wherein the mucoadhesive is present in an amount ranging from 0.01% to 5% (w/v).

6. The pharmaceutical composition of claim 2, wherein the surfactant comprises octoxynol-9.

7. The pharmaceutical composition of claim 2, wherein the surfactant comprises nonoxynol-9.

8. The pharmaceutical composition of claim 6, wherein the surfactant is present in an amount of 0.1% (w/v).

9. The pharmaceutical composition of claim 7, wherein the surfactant is present in an amount of 0.1% (w/v).

10. The pharmaceutical composition according to claim 1 for use in treating a patient with a neurogenic bladder dysfunction, comprising: intravesically instilling to the bladder wall of the patient the above pharmaceutical composition.

11. The pharmaceutical composition for use according to claim 10, wherein the surfactant comprises an alkyl aryl polyether.

12. The pharmaceutical composition for use according to claim 10, wherein the mucoadhesive comprises chitosan.

13. The pharmaceutical composition for use according to claim 10, wherein the surfactant is present in an amount ranging from 0.01% to 0.5% (w/v).

14. The pharmaceutical composition for use according to claim 12, wherein the mucoadhesive is present in an amount ranging from 0.01% to 5% (w/v).

15. The pharmaceutical composition for use according to claim 11, wherein the surfactant comprises octoxynol-9.

## Patentansprüche

1. Pharmazeutische Zusammensetzung, umfassend eine therapeutisch wirksame Menge eines Clostridienderivats und mindestens ein permeabilisierendes Mittel,
wobei das Clostridienderivat ein Botulinumtoxin ist und wobei das mindestens eine permeabilisierende Mittel ein nichtionisches Tensid und ein Mucoadhäsiv, das ein kationisches Polymer ist, umfasst und
wobei das mindestens eine permeabilisierende Mittel in einer wirksamen Menge vorhanden ist, so dass die Durchlässigkeit der Blasenwand für das
Clostridienderivat wesentlich und reversibel erhöht wird.

2. Pharmazeutische Zusammensetzung gemäß Anspruch 1, wobei das Tensid einen Alkylarylpolyether umfasst.

3. Pharmazeutische Zusammensetzung gemäß Anspruch 1, wobei das Mucoadhäsiv Chitosan umfasst.

4. Pharmazeutische Zusammensetzung gemäß Anspruch 1, wobei das Tensid in einer Menge im Bereich von 0,01% bis 0,5% (w/v) vorhanden ist.

5. Pharmazeutische Zusammensetzung gemäß Anspruch 2, wobei das Mucoadhäsiv in einer Menge im Bereich von 0,01% bis 5% (w/v) vorhanden ist.

6. Pharmazeutische Zusammensetzung gemäß Anspruch 2, wobei das Tensid Oxtoxynol-9 umfasst.

7. Pharmazeutische Zusammensetzung gemäß Anspruch 2, wobei das Tensid Nonoxynol-9 umfasst.

8. Pharmazeutische Zusammensetzung gemäß Anspruch 6, wobei das Tensid in einer Menge von 0,1% (w/v) vorhanden ist.

9. Pharmazeutische Zusammensetzung gemäß Anspruch 7, wobei das Tensid in einer Menge von 0,1% (w/v) vorhanden ist.

10. Pharmazeutische Zusammensetzung gemäß Anspruch 1 zur Verwendung bei der Behandlung eines Patienten mit einer neurogenen Blasenfunktionsstörung, umfassend: das intravesikale Instillieren der oben genannten pharmazeutische Zusammensetzung in die Blasenwand des Patienten.

11. Pharmazeutische Zusammensetzung zur Verwendung gemäß Anspruch 10, wobei das Tensid einen Alkylarylpolyether umfasst.

12. Pharmazeutische Zusammensetzung zur Verwendung gemäß Anspruch 10, wobei das Mucoadhäsiv Chitosan umfasst.

13. Pharmazeutische Zusammensetzung zur Verwendung gemäß Anspruch 10, wobei das Tensid in einer Menge im Bereich von 0,01% bis 0,5% (w/v) vorhanden ist.

14. Pharmazeutische Zusammensetzung zur Verwendung gemäß Anspruch 12, wobei das Mucoadhäsiv in einer Menge im Bereich von 0,01% bis 5% (w/v) vorhanden ist.

15. Pharmazeutische Zusammensetzung zur Verwendung gemäß Anspruch 11, wobei das Tensid Octoxynol-9 umfasst.

## Revendications

1. Composition pharmaceutique comprenant une quantité thérapeutiquement efficace d'un dérivé de clostridium et au moins un agent perméabilisant,
dans laquelle le dérivé de clostridium est une toxine botulique, et dans laquelle l'au moins un agent perméabilisant comprend un tensioactif non ionique et un muco-adhésif, qui est un polymère cationique, et
dans laquelle l'au moins un agent perméabilisant est présent en une quantité efficace pour augmenter sensiblement et de façon réversible la perméabilité de la paroi vésicale au dérivé de clostridium.

2. Composition pharmaceutique selon la revendication 1, dans laquelle le tensioactif comprend un polyéther alkyl-aryle.

3. Composition pharmaceutique selon la revendication 1, dans laquelle le muco-adhésif comprend du chitosane.

4. Composition pharmaceutique selon la revendication 1, dans laquelle le tensioactif est présent en une quantité dans la plage de 0,01 % à 0,5 % (p/v).

5. Composition pharmaceutique selon la revendication 2, dans laquelle le muco-adhésif est présent en une quantité dans la plage de 0,01 % à 5 % (p/v).

6. Composition pharmaceutique selon la revendication 2, dans laquelle le tensioactif comprend de l'octoxynol-9.

7. Composition pharmaceutique selon la revendication 2, dans laquelle le tensioactif comprend du nonoxynol-9.

8. Composition pharmaceutique selon la revendication 6, dans laquelle le tensioactif est présent en une quantité de 0,1 % (p/v).

9. Composition pharmaceutique selon la revendication 7, dans laquelle le tensioactif est présent en une quantité de 0,1 % (p/v).

10. Composition pharmaceutique selon la revendication 1 pour son utilisation dans le traitement d'un patient présentant un dysfonctionnement vésical neurogène, comprenant : l'instillation intravésicale dans la paroi vésicale du patient de la composition pharmaceutique ci-dessus.

11. Composition pharmaceutique pour son utilisation selon la revendication 10, dans laquelle le tensioactif comprend un polyéther alkyl-aryle.

12. Composition pharmaceutique pour son utilisation selon la revendication 10, dans laquelle le muco-adhésif comprend du chitosane.

13. Composition pharmaceutique pour son utilisation selon la revendication 10, dans laquelle le tensioactif est présent en une quantité dans la plage de 0,01 % à 0,5 % (p/v).

14. Composition pharmaceutique pour son utilisation selon la revendication 12, dans laquelle le muco-adhésif est présent en une quantité dans la plage de 0,01 % à 5 % (p/v).

15. Composition pharmaceutique pour son utilisation selon la revendication 11, dans laquelle le tensioactif comprend de l'octoxynol-9.
